# EUROPÄISCHE PATENTSCHRIFT

(11) **EP 0 787 711 B1**
(45) Veröffentlichungstag und Bekanntmachung des Hinweises auf die Patenterteilung: **26.04.2000**
(21) Anmeldenummer: 97100843.8
(22) Anmeldetag: 21.01.1997
(51) Int. Cl.: C07C 51/377, C07C 57/42, C07C 57/60, C07C 59/115

(54) **Verfahren zur Herstellung von alpha,beta-ungesättigten Carbonsäuren**
Process for the preparation of alpha,beta-unsaturated carboxylic acids
Procédé de préparation d'acides carboxyliques alpha,bêta-insaturés

(30) Priorität: 31.01.1996 CH 24796
(43) Veröffentlichungstag der Anmeldung: 06.08.1997
(73) Patentinhaber: F. HOFFMANN-LA ROCHE AG, 4070 Basel (CH)
(72) Erfinder: Foricher, Joseph, 68200 Mulhouse (FR); Schmid, Rudolf, 4144 Arlesheim (CH)
(74) Vertreter: Henrich, Werner

(56) Entgegenhaltungen:
- EP-A- 0 268 148
- EP-A- 0 388 739
- EP-A- 0 667 350
- DE-C- 960 814
- US-A- 4 409 397
- OHTA T ET AL: "ASYMMETRIC HYDROGENATION OF UNSATURATED CARBOCYLIC ACIDS CATALYZED BY BINAP-RUTHENIUM(II) COMPLEXES" JOURNAL OF ORGANIC CHEMISTRY, Bd. 52, Nr. 14, 10.Juli 1987, Seiten 3174-3176, XP000577195

## Beschreibung

Die Erfindung betrifft ein neues Verfahren zur Herstellung einer α,β-ungesättigten Säure der allgemeinen Formel 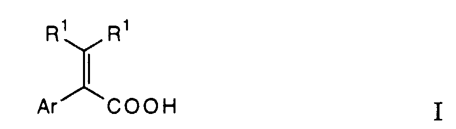 worin R¹ C₁-C₅-Alkyl; Ar ein Arylrest, welcher gegebenenfalls ein- oder mehrfach mit Halogen, Phenyl, C₁-C₅-Alkyl, C₁-C₅-Alkoxy, perfluoriertem C₁-C₅-Alkyl oder perfluoriertem C₁-C₅-Alkoxy substituiert sein kann, bedeuten.

Das erfindungsgemässe Verfahren ist dadurch gekennzeichnet, dass eine Verbindung der allgemeinen Formel 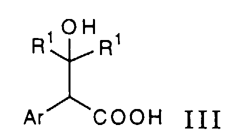 worin R¹ und Ar die obengenannten Bedeutungen haben, in Gegenwart einer starken Säure und gegebenenfalls eines Lösungs-vermittlers bei einer Temperatur von 0-40°C dehydratisiert wird.

Die Verbindungen der Formel I sind an sich bekannt vgl. z.B. US Patentschrift Nr. 4 409 397. Es sind wichtige Zwischenprodukte für die Herstellung von pharmakologisch verwendbaren Endprodukten wie z. B. der Calcium -Antagonist [1S,2S]-2-[2-[[3-(2-Benzimidazolyl)propyl]methylamino]ethyl]-6-fluor-1,2,3,4-tetrahydro-1-isopropyl-2-naphthylmethoxyacetat-dihydrochlorid (Mibefradil) oder auch für die Herstellung von Pestiziden. So können Verbindungen der Formel I direkt durch asymmetrische Hydrierung in Gegenwart eines optisch aktiven Diphosphinkomplexes mit einem Uebergangsmetall der Gruppe VIII, wie beispielsweise Rhodium oder Ruthenium, als Katalysator in die optisch aktiven Säuren der Formel 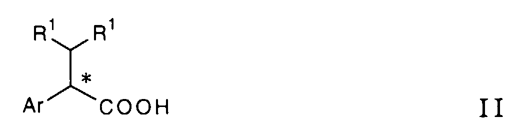 worin R ¹ und Ar die obengenannten Bedeutungen haben, überführt werden.

Durch das erfindungsgemässe Verfahren wird die Herstellung von Verbindungen der Formel I wesentlich vereinfacht und verbessert und es eröffnet sich somit ein direkter Zugang zu den Verbindungen der Formel II, welche bisher vorzugsweise über eine Racematspaltung isoliert wurden. In der Literatur ist zwar beschrieben, dass Verbindungen der Formel I durch Dehydratisierung entsprechender Hydroxyverbindungen hergestellt werden können. So wird beispielsweise in der US Patentschrift Nr. 4 409 397 erwähnt, dass die Dehydratisierung in Gegenwart von p-Toluolsulfonsäure oder KHSO₄ realisiert werden kann.

Die in der US Patentschrift Nr. 4 409 397 empfohlenen Säuren funktionieren in der Tat für die Dehydratisierung von Verbindungen der Formel III, geben jedoch nur geringe Ausbeuten von Verbindungen der Formel I wegen konkurrierender Dehydratisierung zur isomeren Säure der Formel IV und dehydratisierender Decarboxylierung zu Verbindungen der Formel V, wobei letztere als Hauptreaktion abläuft (Schema 1). 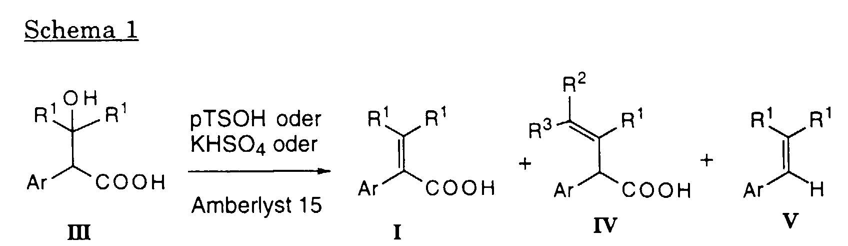 worin R¹ und Ar die obengenannten Bedeutungen haben; und R² und R³ unabhängig voneinander Wasserstoff oder geradkettiges (C₁-C₄)-Alkyl bedeuten, wobei die Reste R² und R³ zusammen höchstens 4 Kohlenstoffatome aufweisen.

Zur Vermeidung dieser Konkurrenzreaktionen kann eine säurekatalysierte Dehydratisierung auf der Esterstufe mit nachfolgender Verseifung durchgeführt werden, dadurch werden aber zwei zusätzliche Stufen benötigt.

### DE 960 814 C offenbart in Beispiel 1 einen Prozess zur Herstellung von 3-Methyl-2-phenyl-penten-(2)-säure durch Dehydratisierung von 2-Phenyl-3-methyl-3-oxy-pentansäure in Gegenwart von konzentrierter Schwefelsäure in Essigsäure als Lösungsmittel bei einer Temperatur von 82°C.

### Der vorliegenden Erfindung liegt die Aufgabe zugrunde, ein vereinfachtes und verbessertes Verfahren zur Herstellung der Verbindungen der Formel I bereitzustellen.

Ueberraschenderweise wurde nun gefunden, dass starke Säuren wie konz. Schwefelsäure (H₂SO₄) oder Polyphosphorsäure die direkte Dehydratisierung von Verbindungen der Formel III unter sehr milden Bedingungen bei Temperaturen von 0-40°C, vorzugsweise bei Temperaturen von 20-25°, sehr effizient bewirken, und dass dabei sowohl die dehydratisierende Decarboxylierung zu Verbindungen der Formel V als auch die Bildung der isomeren Säuren der Formel IV unterdrückt werden. So werden beispielsweise bei der Dehydratisierung mit konz. H₂SO₄ reine ungesättigte Säuren der Formel I in bis zu 99%iger Ausbeute erhalten. Die Schwefelsäure fungiert in dieser Reaktion nicht nur als Säure, sondern auch als wasserbindendes Mittel und als Solvens. Ein weiterer Vorteil ist, dass die Reaktion bei sehr hoher Konzentration durchgeführt werden kann, z.B. mit 2-2,5 Teilen konz. H₂SO₄/1 Teil Hydroxysäure der Formel III. Wird die Hydroxysäure der Formel III in kristalliner Form eingesetzt, so ist es vorteilhaft, zusätzlich einen Lösungsvermittler zu verwenden, welcher auch ein gewisses Lösungsvermögen für H₂SO₄ besitzt, wie beispielsweise CH₂Cl₂, Dioxan oder Essigsäure, vorzugsweise CH₂Cl₂.

Die Dehydratisierung von Verbindungen der Formel III kann in gleicher Weise mit Polyphosphorsäure ebenfalls unter milden Bedingungen bei Temperaturen von 0-40°C, vorzugsweise 20-35°C durchgeführt werden. Andere starke Säuren jedoch, wie beispielsweise HCl, HBr, HI, H₃PO₄ und HCOOH ergeben unter milden Bedingungen keine Reaktion und bei erhöhten Temperaturen entstehen die weiter oben erwähnten Gemische.

Dank der sauberen Dehydratisierung mit konz. H₂SO₄ ergibt sich somit ein überaus vorteilhaftes, kurzes Verfahren zur Herstellung der unge sättigten Säuren der Formel I. Diese werden somit in einem chemisch zweistufigen (technisch einstufigen) Verfahren in bis zu 95% Gesamtausbeute ausgehend von Arylessigsäure erhalten. Ein zusätzlicher Vorteil dieser Synthese ist, dass die ungesättigten Säuren der Formel I in einer sehr hohen Reinheit anfallen, was für die nachfolgende asymmetrische Hydrierung sehr günstig ist.

Die in den Definitionen verwendeten Ausdrücke werden im folgenden erläutert:
"C₁-C₅-Alkyl" bedeutet im Rahmen der vorliegenden Erfindung Methyl, Ethyl, Propyl, Butyl, Pentyl, i-Propyl, 2-Butyl, 2-Pentyl und dergleichen;
"C₁-C₅-Alkoxy" umfasst Gruppen, worin der Alkyl-Rest die obengenannten Bedeutungen hat;
"perfluoriertes C₁-C₅-Alkyl" und "perfluoriertes C₁-C₅-Alkoxy" bedeuten im Rahmen der vorliegenden Erfindung Trifluormethyl, Pentafluorethyl Perfluorpropyl, Perfluorbutyl, Perfluorpentyl, Perfluor-i-propyl, Perfluor-2-butyl, Perfluor-t-butyl, Perfluor-2-pentyl, bzw. Trifluormethoxy, Pentafluorethoxy, Perfluorpropyloxy, Perfluorbutyloxy, Perfluorpentyloxy, Perfluor-i-propyloxy, Perfluor-2-butyloxy oder Perfluor-2-pentyloxy;
"ein Arylrest, welcher gegebenenfalls ein- oder mehrfach mit Halogen, Phenyl, C₁-C₅-Alkyl, C₁-C₅-Alkoxy, perfluoriertem C₁-C₅-Alkyl oder perfluoriertem C₁-C₅-Alkoxy substituiert sein kann" umfasst im Rahmen der vorliegenden Erfindung Phenyl, in o-, m- und/oder p-Stellung fluoriertes, chloriertes, alkyliertes und/oder alkoxyliertes Phenyl wie beispielsweise o-Fluor-, m-Fluor-, p-Fluor-, o-Chlor-, m-Chlor-, p-Chlor-, p-Brom-, p-Methyl-, p-Methoxy-, p-CF₃O-phenyl und dergleichen, sowie 1- oder 2-Naphthyl, welches gegebenenfalls ein- oder mehrfach mit Halogen, C₁-C₅-Alkyl oder C₁-C₅-Alkoxy substituiert sein kann, wie beispielsweise in 6-Stellung fluoriertes, chloriertes, bromiertes, alkyliertes und/oder alkoxyliertes 2-Naphthyl.

Besonders bevorzugte Reste R¹ sind Methyl und Ethyl. Ganz besonders bevorzugte Arylreste Ar sind Phenylreste, welche mit Fluor, Chlor, Brom, Methyl, Methoxy oder Trifluormethoxy, insbesondere in p-Stellung, substituiert sind.

Die Verbindungen der Formel 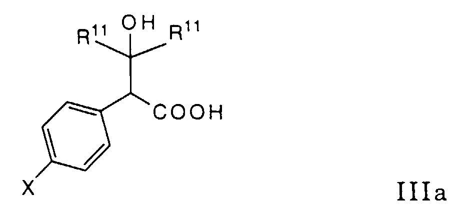 worin R¹¹ Methyl oder Ethyl; und X Fluor, Chlor oder Brom bedeutet, sind neu und ebenfalls Gegenstand der Erfindung.

Die Verbindungen der Formel III und somit auch die Verbindungen der Formel IIIa können in an sich bekannter Weise ausgehend von den entsprechenden Arylessigsäuren der Formel VI hergestellt werden, siehe Schema 2. Die Herstellung beinhaltet beispielsweise die Ueberführung der entsprechenden Arylessigsäuren in die Di-Anionen mit 2 Aequivalenten einer starken Base und nachfolgende Reaktion mit Aceton. Als starke Basen kommen in Frage Lithium- und Natriumamide, inbesondere Lithiumdialkylamide, Organolithiumverbindungen oder Organomagnesiumverbindungen, insbesondere Grignard-Reagenzien. Aus wirtschaftlichen Gründen sind die Grignard-Reagenzien (RMgX, X = Cl, Br) zu bevorzugen, insbesondere MeMgCl, EtMgCl oder iPropMgCl. Allenfalls kann 1 Aequivalent der starken Base auch durch NaH zur Bildung des Na-Carboxylates ersetzt werden, oder es kann ein Alkali- oder Erdalkalisalz der Säure direkt eingesetzt werden, um 1 Aequivalent der starken Base zu sparen.

Die Hydroxysäuren der Formel III können, falls erwünscht, rein isoliert und kristallisiert werden; vorteilhafterweise werden sie jedoch direkt als ölige Rohprodukte der Dehydratisierung unterworfen. 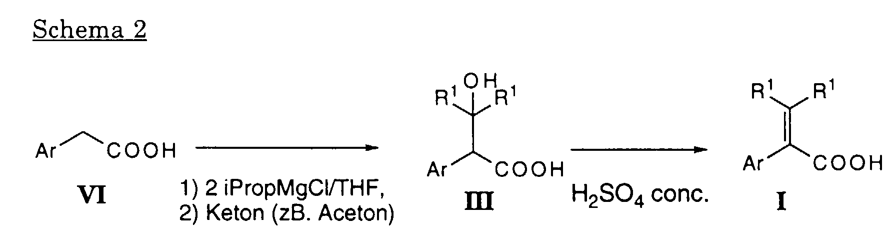 worin R¹ und Ar die obengenannten Bedeutungen haben.

Ebenfalls Gegenstand der vorliegenden Erfindung ist die Herstellung von optisch aktiven Säuren der Formel 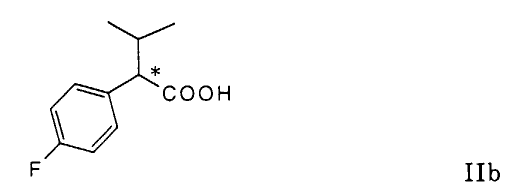 durch enantioselektive Hydrierung der Verbindung der Formel 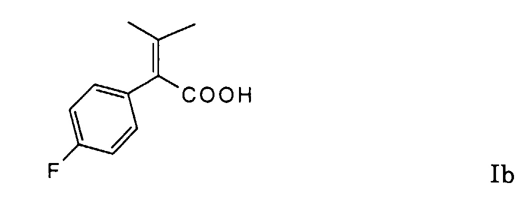 in Gegenwart eines optisch aktiven Rutheniumdiphosphinkomplexes, z.B. eines Ruthenium-(R)-(6,6'-Dimethylbiphenyl-2,2'-diyl)bis(diphenylphosphin)-, eines Ruthenium-(R)-(6,6'-Dimethoxybiphenyl-2,2'-diyl)bis(diphenylphosphin)- oder eines Ruthenium-[(R)-1-{(1S,2R)-1',2-Bis-diphenylphosphanyl-ferrocenyl}-ethyl]-methyl-(2-piperidin-1-yl-ethyl)-amin-Komplexes als Katalysator, wobei die Verbindung Ib durch Dehydratisierung der Verbindung der Formel IIIb hergestellt wird.

Die Verbindung (S)-2-(p-Fluorphenyl)-3-methyl-buttersäure wird mit Methylenchlorid, Thionylchlorid, Aluminiumtrichlorid und Ethylen in das entsprechende 6-fluor-1,2,3,4 tetrahydro-1-isopropyl-2-naphthalin-on umgewandelt, das mit tert. Butylacetat in den entsprechenden Tetrahydronaphthalin-hydroxyester überführt wird, der mit Benzimidazolpropylamin in an sich bekannter Weise, gemäss der EP-B-0 268 148 in [1S,2S]-2-[2-[[3-(2-Benzimidazolyl)propyl]methyl-amino]ethyl]-6-fluor-1,2,3,4-tetrahydro-1-isopropyl-2-naphthylmethoxyacetat-dihydrochlorid (Mibefradil) überführt wird.

Die nachfolgenden Beispiele sollen den Gegenstand der vorliegenden Erfindung weiter erläutern, sie stellen jedoch in keiner Weise eine Beschränkung dar. Die in den Beispielen verwendeten Abkürzungen haben folgende Bedeutung:
- (R)-BIPHEMP =: (R)-(6,6'-Dimethylbiphenyl-2,2'-diyl)bis(diphenyl phosphin)
- (R)-MeOBIPHEP =: (R)-(6,6'-Dimethoxybiphenyl-2,2'-diyl)bis(diphenyl phosphin)
- (R,S)-BPPFA-EPIP =: [(R)-1-{(1S,2R)-1',2-Bis-diphenylphosphanyl-ferro cenyl}-ethyl]-methyl-(2-piperidin-1-yl-ethyl)-amin
- OAc =: Acetat
- COD =: 1,5-Cyclooctadien
- RT =: Raumtemperatur

### Beispiel 1

### Herstellung von 2-(p-Fluorphenyl)-3-methylcrotonsäure (ohne Isolierung der Hydroxysäure)

a) In einem 1.5-l-Vierhalssulfierkolben mit Rückflusskühler, mechanischem Rührer, 500-ml-Tropftrichter, Thermometer und einer Vorrichtung zur Inertbegasung wurden unter Argon und Rühren 24.3 g (1.00 mol) Magnesiumspäne in 50 ml Tetrahydrofuran suspendiert. Nach Zutropfen von 0.5 ml 1,2-Dibromäthan wurde eine Lösung von 86.4 g (1.10 mol) Isopropylchlorid in 225 ml Tetrahydrofuran innerhalb von 90 Minuten zugetropft, wobei die Reaktionstemperatur bei ca. 30° gehalten wurde. Die entstandene dunkelgraue Suspension wurde noch 18 Stunden bei RT gerührt. Zum Gemisch wurde dann eine Lösung von 73.0 g (0.473 mol) p-Fluorphenylessigsäure in 150 ml Tetrahydrofuran innerhalb von 90 Minuten zugetropft, wobei die Reaktionstemperatur bei ca. 25° gehalten wurde, danach wurde die Suspension auf 35-40° erwärmt und noch 1 Stunde bei dieser Temperatur gerührt. Nach Abkühlung wurden 30.0 g (0.516 mol) Aceton bei ca. 25° innerhalb von 30 Minuten zugetropft und anschliessend noch 1 Stunde bei 35-40° gerührt. Die Reaktionsmischung wurde unter Eisbadkühlung bei < 30° mit 350 ml 14.2% Schwefelsäure versetzt. Die wässrige Phase wurde abgetrennt, mit 200 ml Tetrahydrofuran extrahiert, und die organischen Phasen wurden vereinigt und am Rotationsverdampfer bei 50° eingedampft. Es wurden 110 g rohe 2-(p-Fluorphenyl)-3-hydroxy-3-methylbuttersäure als dunkles viskoses Oel erhalten.
b) Dieses Öl (in einem 1-l-Rundkolben, ausgerüstet mit Magnetrührer, Tropftrichter und Thermometer) wurde unter Eisbadkühlung innerhalb 5-10 Minuten mit 240 g konz. Schwefelsäure versetzt, wobei die Reaktionstemperatur 20° nicht überstieg. Das resultierende Reaktionsgemisch wurde 45 Minuten bei 20° gerührt, dann unter gutem Rühren auf 1 kg Eis/Wasser-Gemisch gegossen. Der rosafarbene Niederschlag wurde abgenutscht, 3mal mit je 100 ml Wasser und 2mal mit je 200 ml Hexan gewaschen und schliesslich unter Erwärmen auf 50° in 500 ml Methanol aufgenommen. Die erhaltene rosafarbene Lösung wurde mit 8 g Entfärbungskohle während 30 Minuten bei 50° gerührt, filtriert und eingeengt. Der feste Rückstand wurde in 500 ml Hexan bei 50° aufgeschlämmt, nach Abkühlung auf RT genutscht, 2mal mit je 50 ml Hexan gewaschen und 1 Stunde bei 1 mbar getrocknet. Es wurden 88.0 g 2-(p-Fluorphenyl)-3-methylcrotonsäure als weisses Pulver vom Smp. 124-126° erhalten; GC-Reinheit 99.8 Fl%; Ausbeute 95.7% bezogen auf p-Fluorphenylessigsäure.

### Beispiel 2

### Herstellung von 2-(p-Fluorphenyl)-3-methylcrotonsäure mit Kristallisation des Zwischenproduktes 2-(p-Fluorphenyl)-3-hydroxy-3-methylbuttersäure

a) Die aus einer zu 1 a) analog durchgeführten Reaktion erhaltene Lösung von 2-(p-Fluorphenyl)-3-hydroxy-3-methylbuttersäure (ex 0.473 mol p-Fluorphenylessigsäure) in Tetrahydrofuran wurde über MgSO₄ getrocknet, filtriert und eingedampft. Der viskose Rückstand wurde bei 50° in 100 ml Toluol gelöst, und die Lösung wurde während 15 Minuten bei 50° mit Entfärbungskohle gerührt, filtriert und am Rotationsverdampfer bei 50° aufkonzentriert. Nach Abdestillation von ca. 30 ml Toluol wurde bei 50° mit 300 ml Hexan versetzt. Nach Abkühlen, zuletzt im Eisbad, wurde das Kristallisat genutscht, 2mal mit je 50 ml Hexan gewaschen und bei 15 Torr/80° während 1 Stunde getrocknet. Es wurden 97.0 g (96.5%) 2-(p-Fluorphenyl)-3-hydroxy-3-methylbuttersäure als weisse Kristalle vom Smp. 86-88° erhalten; GC-Reinheit 98%.
b) Eine Lösung von 96.5 g 2-(p-Fluorphenyl)-3-hydroxy-3-methylbuttersäure in 200 ml CH₂Cl₂ wurde in einem Eisbad gekühlt und dann innerhalb von 30 Minuten tropfenweise mit 240 g konz. Schwefelsäure versetzt, wobei die Reaktionstemperatur auf ≤ 20° gehalten wurde. Das CH₂Cl₂ wurde dann am Rotationsverdampfer bei 20° entfernt. Die gelbliche Lösung wurde noch 45 Minuten bei 20° gerührt und dann unter gutem Rühren auf 1 kg Eis/ Wasser-Gemisch gegossen. Der weisse Niederschlag wurde genutscht, 3mal mit je 100 ml Wasser und 2mal mit je 100 ml Hexan gewaschen und schliesslich im Trockenschrank während 16 Stunden bei 40° und 2 Stunden bei 100° getrocknet. Es wurden 87.5 g 2-(p-Fluorphenyl)-3-methylcrotonsäure als weisses Pulver vom Smp. 124-126° erhalten; GC-Reinheit 99.9%; Ausbeute 95.6% bezogen auf p-Fluorphenylessigsäure.

Zur weiteren Reinigung wurden 76.7 g dieses Materials in 300 ml CH₂Cl₂ bei 50° gelöst. Die Lösung wurde mit 5 g Magnesiumsulfat und 2 g Entfärbungskohle versetzt, gerührt und nach Abkühlen filtriert. Das farblose Filtrat wurde am Rotationsverdampfer unter Argon bei 50° konzentriert. Nach Destillation von ca. 300 ml CH₂Cl₂ wurde bei 50° mit 100 ml Hexan versetzt, weitere 75 ml Lösungsmittel abdestilliert und nochmals mit 75 ml Hexan versetzt. Nach Abkühlen, zuletzt im Eisbad, wurde das Kristallisat abgenutscht, 2mal mit je 50 ml Hexan gewaschen und im Trockenschrank während 1 Stunde bei 50° getrocknet. Es wurden 75.4 g 2-(p-Fluorphenyl)-3-methylcrotonsäure als weisses Pulver vom Smp. 124-126° erhalten; GC-Reinheit 99.95%. Die Kristallisationsausbeute beträgt 98.3%, die Gesamtausbeute bezogen auf 2-(p-Fluorphenyl)-3-hydroxy-3-methylbuttersäure 97% und bezogen auf p-Fluorphenylessigsäure 94%.

### Beispiel 3

### Ethylmagnesiumchlorid als Base

In Analogie zu Beispiel 1a) wurden 70.0 g (0.454 mol) p-Fluorphenylessigsäure mit Ethylmagnesiumchlorid als Base umgesetzt. Es wurden 104 g rohe 2-(p-Fluorphenyl)-3-hydroxy-3-methylbuttersäure als dunkles Öl erhalten.

### Beispiel 4

### Methylmagnesiumchlorid als Base

In Analogie zu Beispiel 1a) wurden 52.5 g (0.34 mol) p-Fluorphenylessigsäure mit Methylmagnesiumchlorid als Base umgesetzt. Es wurden 63.6 g rohe 2-(p-Fluorphenyl)-3-hydroxy-3-methylbuttersäure als dunkles Öl erhalten.

### Beispiel 5

### Dehydratisierung mit Polyphosphorsäure

In einem 350-ml-Sulfierkolben wurden 90 g Polyphosphorsäure vorgelegt. Dann wurde tropfenweise mit einer Lösung von 10.0 g (47 mmol) 2-(p-Fluorphenyl)-3-hydroxy-3-methylbuttersäure in 70 ml CH₂Cl₂ bei 20-25° versetzt. Das viskose, sich gelb färbende Gemisch wurde 2 Stunden gerührt, wobei die Temperatur bis auf 35° anstieg. Nach Hydrolyse mit Eiswasser wurde über Nacht stehen gelassen, dann mit Äther versetzt. Die organische Phase wurde abgetrennt, 3mal mit Wasser gewaschen, über MgSO₄ getrocknet, filtriert und das Filtrat eingeengt. Der Rückstand wurde in CH₂Cl₂ gelöst, die Lösung mit Hexan versetzt und am Rotationsverdampfer konzentriert bis zum Eintreten der Kristallisation. Nach 1 Stunde Stehen bei RT wurden die Kristalle genutscht und mit kaltem Hexan gewaschen. Es wurden 67 g (73%) 2-(p-Fluorphenyl)-3-methylcrotonsäure als weisses Pulver erhalten.

### Beipiel 6

### Herstellung von 2-(p-Chlorphenyl)-3-methylcrotonsäure

a) 2-(p-Chlorphenyl)-3-hydroxy-3-methylbuttersäure. - In einem 1.5-l-Vierhalssulfierkolben mit Rückflusskühler, mechanischem Rührer, 500-ml-Tropftrichter, Thermometer und einer Vorrichtung zur Inertbegasung wurden unter Argon und Rühren 24.3 g (1.00 mol) Magnesiumspäne in 50 ml Tetrahydrofuran suspendiert. Nach Zutropfen von 0.5 ml 1,2-Dibromäthan wurde eine Lösung von 86.4 g (1.10 mol) Isopropylchlorid in 225 ml Tetrahydrofuran innerhalb von 90 Minuten zugetropft, wobei die Reaktionstemperatur auf 30-35° gehalten wurde. Die erhaltene dunkelgraue Suspension wurde noch 18 Stunden bei RT gerührt. Dann wurde innerhalb von 1 Stunde eine Lösung von 80.6 g (0.473 mol) p-Chlorphenylessigsäure in 150 ml Tetrahydrofuran zugetropft, wobei die Reaktionstemperatur 25° nicht überstieg. Die erhaltene viskose gelbliche Suspension wurde noch 1 Stunde auf 35° erwärmt. Dann wurden bei 25° innerhalb von 30 Minuten 30.0 g (0.516 mol) Aceton zugetropft, und anschliessend wurde noch 1 Stunde auf 35° erwärmt. Die Reaktionsmischung wurde unter Eisbadkühlung bei < 30° mit 350 ml 14.2% Schwefelsäure versetzt. Die organische Phase wurde abgetrennt, die wässrige Phase mit 100 ml Tetrahydrofuran extrahiert, und die vereinigten organischen Extrakte wurden am Rotationsverdampfer bei 50° zur Trockne eingedampft. Es wurden 120.9 g rohe 2-(p-Chlorphenyl)-3-hydroxy-3-methylbuttersäure als rohes Öl erhalten. Eine Probe zur Analyse wurde durch Kristallisation aus EtOH/Wasser 1:2 als weisse Kristalle erhalten; Smp. 65°.
b) 2-(p-Chlorphenyl)-3-methylcrotonsäure. - In einem 1-1-Rundkolben mit Magnetrührer, Thermometer und Tropftrichter wurden die obig erhaltenen 120.9 g rohe 2-(p-Chlorphenyl)-3-hydroxy-3-methylbuttersäure in 200 ml CH₂Cl₂ gelöst. Die Lösung wurde unter Eisbadkühlung mit 240 g konz. Schwefelsäure versetzt, wobei die Reaktionstemperatur 20° nicht überstieg. Das CH₂Cl₂ wurde dann am Rotationsverdampfer bei 20° entfernt. Das zurückbleibende dunkle Oel wurde noch 30 Minuten bei 50° gerührt und dann unter gutem Rühren auf 1 kg Eis gegossen. Der rosafarbene Niederschlag wurde genutscht, 3mal mit je 100 ml Wasser gewaschen und in 400 ml CH₂Cl₂ aufgenommen. Die Lösung wurde mit 20 g Magnesiumsulfat und 1 g Entfärbungskohle gerührt und über 200 g Speedex filtriert, wobei mit 100 ml CH₂Cl₂ nachgewaschen wurde. Das Filtrat wurde am Rotationsverdampfer konzentriert. Nach Abdestillation von ca. 250 ml Lösungsmittel wurde mit 250 ml Hexan versetzt. Dieser Vorgang wurde noch 2mal wiederholt, wobei Kristallisation eintrat. Die Kristalle wurden genutscht, mit 100 ml Hexan gewaschen und bei 0.1 mbar getrocknet. Es wurden 76.3 g 2-(p-Chlorphenyl)-3-methylcrotonsäure als weisse Kristalle vom Smp. 144-145° erhalten; Ausbeute 76.6% bezogen auf p-Chlorphenylessigsäure.

### Beispiel 7

### 7.1. Asymmetrische Hydrierung von 2-(p-Fluorphenyl)-3-methylcrotonsäure

a) Ruthenium-katalysierte Hydrierung. - In einer Glove Box (O₂-Gehalt <1 ppm) wurde eine Katalysatorlösung durch Lösen von 0.418 g (0.544 mmol) Ru(OAc)₂[(R)-BIPHEMP] in 100 ml Methanol hergestellt und 10 Minuten bei RT gerührt. Dann wurden 211.2 g (1.0875 mol) 2-(p-Fluorphenyl)-3-methylcrotonsäure und 700 ml Methanol in einem 2-1-Autoklaven vorgelegt und die oben hergestellte Katalysatorlösung zugegeben. Der Autoklav wurde verschlossen und die Hydrierung unter Rühren bei 10° und einem Druck von 150-180 bar durchgeführt. Nach 24 Stunden betrug der Umsatz 100%. Die Hydrierlösung wurde bei 50°/200 mbar eingedampft und der Rückstand bei 108-110°/0.01 mbar destilliert. Es wurden 208.1 g (97.5%) (S)-2-(p-Fluorphenyl)-3-methylbuttersäure als farbloses Öl erhalten, welches bei RT erstarrte; Smp. 53-56°; 96.6% ee.
b) In einer Glove Box (O₂-Gehalt < 1 ppm) wurde bei RT eine Katalysatorlösung durch Lösen von 0.124 g (0.155 mmol) Ru(OAc)₂[(R)-MeOBIPHEP] in 50 ml Methanol hergestellt. Dann wurden 30.0 g (154.5 mmol) 2-(p-Fluorphenyl)-3-methylcrotonsäure und 38 ml Methanol in einem 185-ml-Autoklaven vorgelegt und die oben hergestellte Katalysatorlösung zugegeben. Der Autoklav wurde verschlossen und die Hydrierung unter Rühren bei 20° und einem konstanten Druck von 180 bar durchgeführt. Nach 6 Stunden betrug der Umsatz 100%. Die Hydrierlösung wurde bei 50°/20 mbar eingedampft und der Rückstand im Kugelrohrofen bei 125°/0.2 mbar destilliert. Man erhielt 28.04 g (92.5%) (S)-2-(p-Fluorphenyl)-3-methylbuttersäure als farbloses Oel, welches bei RT auskristallisierte; chemische Reinheit 99.9 GC-Fl%; 90% ee.
c) In einer Glov-Box (O₂-Gehalt < 1 ppm) wurden in einem 30 ml-Autoklav bei RT 1.0 g (5.15 mMol) 2-(p-Fluorphenyl)-3-methylcrotonsäure und 0.52 g (5.15 mMol) Triethylamin in 6 ml Methanol suspendiert und eine Lösung von 4.1 mg (0.0051 mMol) Ru(OAc)₂((R)-MeOPIPHEP) in 5 ml Methanol als Katalysator zugegeben. Der Autoklav wurde verschlossen und die Hydrierung unter Rühren bei 20° und einem Anfangsdruck von 200 bar durchgeführt. Während der Reaktion nahm der Druck auf ca. 190 bar ab. Nach 21 Stunden Reaktionszeit wurde eine Probe der Hydrierlösung bei 50°/20 mbar eingedampft und analysiert. Man erhielt (S)-2-(p-Fluorphenyl)-3-methylbutansäure mit einem ee von 93%.
d)-l) In zu Beispiel c) analoger Weise wurden die Hydrierungen unter Zusatz der in Tabelle 1 aufgeführten Basen durchgeführt:

**Tabelle 1**

| Beispiel | Base | Umsatz [%] | ee [%] (S) |
|---|---|---|---|
| 7.1.d) | Tributylamin | 100 | 93 |
| 7.1.e) | Diethylamin | 100 | 93 |
| 7.1.f) | (R)-1-Phenyl-ethylamin | 70 | 92 |
| 7.1.g) | (S)-1-Phenyl-ethylamin | 93 | 92 |
| 7.1.h) | Pyrrolidin | 100 | 93 |
| 7.1.i) | Diisopropylamin | 100 | 93 |
| 7.1.j) | N-Ethyldiisopropylamin | 100 | 92 |
| 7.1.k) | Ethanolamin | 50 | 92 |
| 7.1.l) | 25proz. NH₄OH-Lösung | 100 | 91 |

m) Rhodium-katalysierte Hydrierung. - In einer Glove Box (O₂-Gehalt < 1 ppm) wurde eine Katalysatorlösung durch Lösen von 0.01045 g (0.0257 mmol) [Rh(COD)₂]BF₄ und 0.01861 g (0.0257 mmol) (R,S)-BPPFA-EPIP in 20 ml Tetrahydrofuran hergestellt und 15 Minuten bei RT gerührt. Dann wurden 5.0 g (25.75 mmol) 2-(p-Fluorphenyl)-3-methylcrotonsäure, 13 ml Methanol und 33 ml Tetrahydrofuran in einem 185-ml-Autoklaven vorgelegt und die oben hergestellte Katalysatorlösung zugegeben. Der Autoklav wurde verschlossen und die Hydrierung unter Rühren bei 20° und einem konstanten Druck von 50 bar durchgeführt. Nach 24 Stunden betrug der Umsatz 100%. Die Hydrierlösung wurde bei 50°/200 mbar eingedampft und der Rückstand bei 108-110°/0.01 mbar destilliert. Es wurden 4.8 g (97%) (S)-2-(p-Fluorphenyl)-3-methylbuttersäure als farbloses Oel erhalten, welches bei RT erstarrte; 98.2% ee.

### 7.2. Asymmetrische Hydrierung von 2-(p-Chlorphenyl)-3-methylcrotonsäure

a) Ruthenium-katalysierte Hydrierung. - In einer Glove Box (O₂-Gehalt <1 ppm) wurde eine Katalysatorlösung durch Lösen von 0.0914 g (0.119 mmol) Ru(OAc)₂[(S)-BIPHEMP] in 50 ml Methanol hergestellt und 15 Minuten bei 20° gerührt. Dann wurden 5.0 g (23.74 mmol) 2-(p-Chlorphenyl)-3-methylcrotonsäure und 23 ml Methanol in einem 185-ml-Autoklaven vorgelegt und die oben hergestellte Katalysatorlösung zugegeben. Der Autoklav wurde verschlossen und die Hydrierung unter Rühren bei 20° und einem konstanten Druck von 60 bar durchgeführt. Nach 4 Stunden betrug der Umsatz 100%. Die Hydrierlösung wurde bei 50°/20 mbar eingedampft und der Rückstand im Kugelrohrofen bei 150°/0.2 mbar destilliert. Man erhielt 4.6 g (92%) (R)-2-(p-Chlorphenyl)-3-methylbuttersäure als farbloses Oel, welches bei RT auskristallisierte; chemische Reinheit 98.8 GC-Fl%; 90.5% ee; [a]₅₈₉ = -42.3° (c = 1, Methanol).
b) Rhodium-katalysierte Hydrierung. - In einer Glove Box (O₂-Gehalt <1 ppm) wurde eine Katalysatorlösung durch Lösen von 0.0482 g (0.119 mmol) [Rh(COD)₂]BF₄ und 0.0858 g (0.119 mmol) (R,S)-BPPFA-EPIP in 20 ml Tetrahydrofuran hergestellt und 15 Minuten bei 20° gerührt. Dann wurden 5.0 g (23.74 mmol) 2-(p-Chlorphenyl)-3-methylcrotonsäure, 13 ml Methanol, 33 ml Tetrahydrofuran in einem 185-ml-Autoklaven vorgelegt und die oben hergestellte Katalysatorlösung zugegeben. Der Autoklav wurde verschlossen und die Hydrierung unter Rühren bei 20° und einem konstanten Druck von 50 bar durchgeführt. Nach 18 Stunden betrug der Umsatz 100%. Die Hydrierlösung wurde bei 50°/20 mbar eingedampft und der Rückstand im Kugelrohrofen bei 150°/0.2 mbar destilliert. Man erhielt 4.8 g (96%) (S)-2-(p-Chlorphenyl)-3-methylbuttersäure als farbloses Oel, welches bei RT auskristallisierte; chemische Reinheit 99.9 GC-Fl%; 97.6% ee.

## Patentansprüche

1. Verfahren zur Herstellung von Verbindungen der allgemeinen Formel 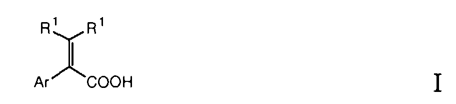 worin R¹ C₁-C₅-Alkyl; Ar einen Arylrest, welcher gegebenenfalls ein- oder mehrfach mit Halogen, Phenyl, C₁-C₅-Alkyl, C₁-C₅-Alkoxy, perfluoriertem C₁-C₅-Alkyl oder perfluoriertem C₁-C₅-Alkoxy substituiert sein kann, bedeuten,
dadurch gekennzeichnet, dass eine Verbindung der allgemeinen Formel 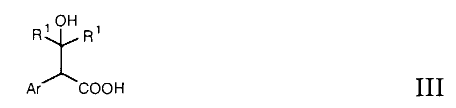 worin R¹ und Ar die obengenannten Bedeutungen haben,
in Gegenwart einer starken Säure und gegebenenfalls eines Lösungs-vermittlers bei einer Temperatur von 0-40°C dehydratisiert wird.

2. Verfahren gemäss Anspruch 1, dadurch gekennzeichnet, dass die Dehydratisierung in Gegenwart von konzentrierter Schwefelsäure oder konz. Polyphosphorsäure durchgeführt wird.

3. Verfahren gemäss einem der Ansprüche 1 bis 2, dadurch gekennzeichnet, dass als Lösungsvermittler Dioxan, Essigsäure oder Methylenchlorid verwendet wird.

4. Verfahren gemäss einem der Ansprüche 1 bis 3, dadurch gekennzeichnet, dass die Dehydratisierung bei 20-25°C durchgeführt wird.

5. Verfahren gemäss einem der Ansprüche 1 bis 4, dadurch gekennzeichnet, dass der Arylrest einen mit Fluor, Chlor, Brom, Methyl, Methoxy oder Trifluormethoxy, vorzugsweise in p-Stellung, substituierten Phenylrest bedeutet.

6. Verfahren zur Herstellung von Verbindungen der Formel 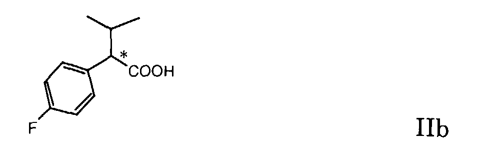 dadurch gekennzeichnet, dass eine Verbindung der allgemeinen Formel 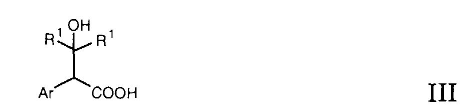 worin R¹ Methyl und Ar p-Fluorphenyl bedeuten,
nach einem Verfahren der Ansprüche 1 bis 4 dehydratisiert und die erhaltene Verbindung der Formel 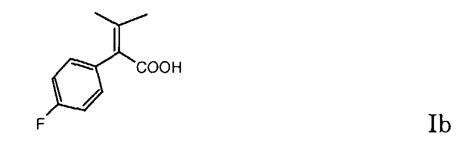 in Gegenwart eines optisch aktiven Rutheniumdiphosphin Komplexes als Katalysator in eine optisch aktive Säure der Formel IIb übergeführt wird.

7. Verbindungen der Formel 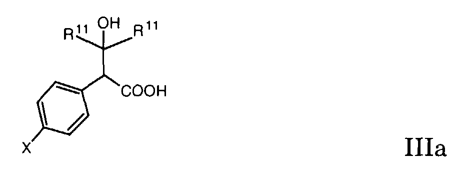 worin R¹¹ Methyl oder Ethyl; und X Fluor, Chlor oder Brom bedeutet.

8. Verwendung des Verfahrens nach einem der Ansprüche 1 bis 6 zur Herstellung von [1S,2S]-2-[2-[[3-(2-Benzimidazolyl)propyl]methyl-amino]ethyl]-6-fluor-1,2,3,4-tetrahydro-1-isopropyl-2-naphthylmethoxyacetatdihydrochlorid (Mibefradil).

## Claims

1. A process for the manufacture of compounds of the general formula 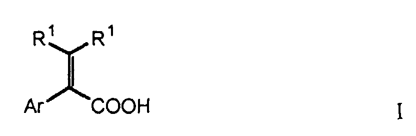 wherein R¹ signifies C₁-C₅-alkyl; Ar signifies an aryl residue which can be optionally substituted by one or more halogen, phenyl, C₁-C₅-alkyl, C₁-C₅-alkoxy, perfluorinated C₁-C₅-alkyl or perfluorinated C₁-C₅-alkoxy substituents,
characterized by dehydrating a compound of the general formula 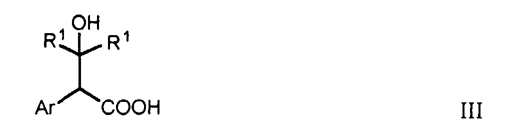 wherein R¹ and Ar have the significances set forth above,
in the presence of a strong acid and, if desired, a solubilizer at a temperature of 0-40°C.

2. A process according to claim 1, characterized in that the dehydration is carried out in the presence of concentrated sulphuric acid or conc. polyphosphoric acid.

3. A process according to either of claims 1 to 2, characterized in that dioxan, acetic acid or methylene chloride is used as the solubilizer.

4. A process according to any one of claims 1 to 3, characterized in that the dehydration is carried out at 20-25°C.

5. A process according to any one of claims 1 to 4, characterized in that the aryl residue signifies a phenyl residue substituted, preferably in the p-position, with fluorine, chlorine, bromine, methyl, methoxy or trifluoromethoxy.

6. A process for the manufacture of compounds of the formula 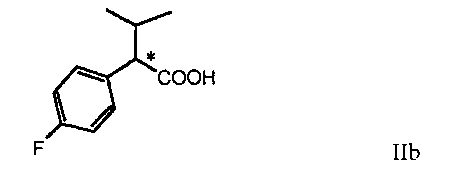 characterized by dehydrating a compound of the general formula 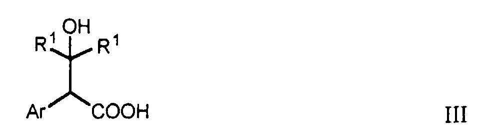 wherein R¹ signifies methyl and Ar signifies p-fluorophenyl,
according to a process of claims 1 to 4 and converting the resulting compound of the formula 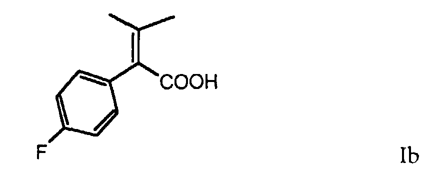 into an optically active acid of formula IIb in the presence of an optically active ruthenium diphosphine complex as the catalyst.

7. Compounds of the formula 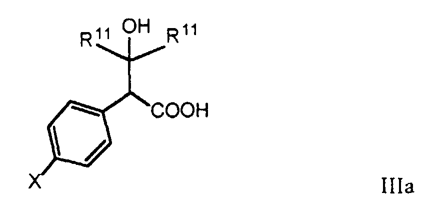 wherein R¹¹ signifies methyl or ethyl; and X signifies fluorine, chlorine or bromine.

8. The use of the process according to any one of claims 1 to 6 for the manufacture of [1S,2S]-2-[2-[[3-(2-benzimidazolyl)propyl]methylamino]ethyl]-6-fluoro-1,2,3,4-tetrahydro-1-isopropyl-2-naphthylmethoxyacetate dihydrochloride (mibefradil).

## Revendications

1. Procédé de préparation de composés de formule générale 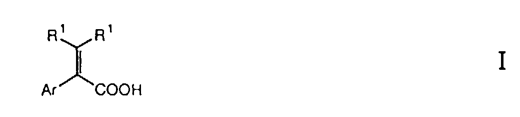 dans laquelle R¹ représente un alkyle en C₁ à C₅ ; Ar représente un radical aryle, qui peut le cas échéant être mono- ou polysubstitué par un halogène, un phényle, un alkyle en C₁ à C₅, un alcoxy en C₁ à C₅, un alkyle en C₁ à C₅ perfluoré ou un alcoxy en C₁ à C₅ perfluoré,
caractérisé en ce qu'on déshydrate à une température de 0 à 40°C un composé de formule générale 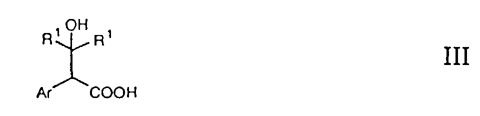 dans laquelle R¹ et Ar ont les significations ci-dessus,
en présence d'un acide fort et le cas échéant d'un tiers solvant.

2. Procédé selon la revendication 1, caractérisé en ce qu'on procède à la déshydratation en présence d'acide sulfurique concentré ou d'acide polyphosphorique concentré.

3. Procédé selon les revendications 1 à 2, caractérisé en ce qu'on utilise comme tiers solvant le dioxanne, l'acide acétique ou le chlorure de méthylène.

4. Procédé selon les revendications 1 à 3, caractérisé en ce qu'on conduit la déshydratation entre 20 et 25°C.

5. Procédé selon les revendications 1 à 4, caractérisé en ce que le radical aryle représente un radical phényle substitué par un fluor, un chlore, un brome, un méthyle, un méthoxy ou un trifluorométhoxy, de préférence en position p.

6. Procédé de préparation de composés de formule 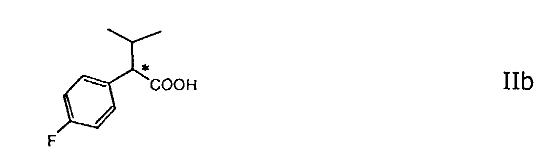 caractérisé en ce qu'on déshydrate un composé de formule générale 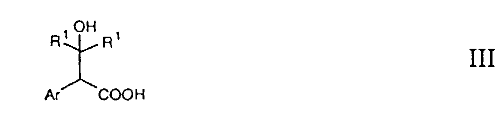 dans laquelle R¹ représente un méthyle et Ar un p-fluorophényle, d'après le procédé des revendications 1 à 4,
et en ce qu'on transforme le composé obtenu de formule 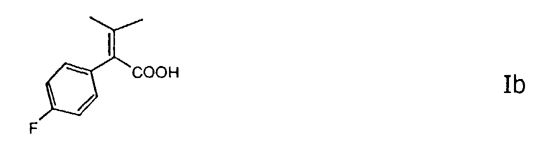 en présence d'un complexe ruthénium-diphosphine optiquement actif comme catalyseur, en un acide de formule IIb optiquement actif.

7. Composé de formule 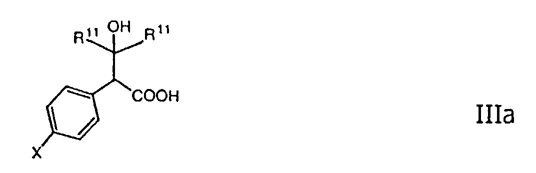 dans laquelle R¹¹ représente un méthyle ou un éthyle ; et X représente un fluor, un chlore ou un brome.

8. Utilisation du procédé selon l'une des revendications 1 à 6 pour préparer du dichlorhydrate de méthoxyacétate de [1S,2S]-2-[2-[[3-(2-benzimidazolyl)propyl]méthylamino]éthyl]-6-fluoro-1,2,3,4-tétrahydro-1-isopropyl-2-naphtyle (Mibefradil).
